# EUROPEAN PATENT SPECIFICATION

(11) **EP 3 250 047 B1**
(45) Date of publication and mention of the grant of the patent: **19.03.2025**
(21) Application number: 16701868.8
(22) Date of filing: 22.01.2016
(51) Int. Cl.: A23J 3/22, A23L 13/00, A23L 13/40, A23L 29/00, A23L 31/00, A23L 33/175, A23L 33/185, A23J 1/00

(54) **AN EDIBLE FORMULATION COMPRISING EDIBLE FUNGAL PARTICLES AND METHOD OF MAKING AN EDIBLE FORMULATION**
ESSBARE FORMULIERUNG AUS ESSBAREN PILZPARTIKELN UND VERFAHREN ZUR HERSTELLUNG EINER ESSBAREN FORMULIERUNG
FORMULATION COMESTIBLE COMPRENANT DES PARTICULES FONGIQUES COMESTIBLES ET PROCÉDÉ DE FABRICATION D'UNE FORMULATION COMESTIBLE

(30) Priority: 27.01.2015 GB 201501320
(43) Date of publication of application: 06.12.2017
(73) Proprietor: Marlow Foods Limited, Stokesley, Yorkshire TS9 7AB (GB)
(72) Inventor: FINNIGAN, Timothy, Hutton Rudby Yorkshire TS15 0JZ (GB); AKINTOYE, Muyiwa, Guisborough Cleveland TS14 8EZ (GB); MOUSAVI, Reza, Middlesbrough Yorkshire TS7 9PD (GB)
(74) Representative: Appleyard Lees IP LLP
(86) International application number: PCT/GB2016/050144
(87) International publication number: WO 2016/120594

(56) References cited:
- EP-A1- 0 986 960
- EP-A1- 1 133 926
- WO-A1-02/089589
- WO-A1-02/090527
- WO-A1-2004/026039
- WO-A1-2014/110539
- WO-A1-91/17669
- WO-A1-99/55165
- WO-A2-02/089604
- WO-A2-03/007728
- DE-A1- 2 848 699
- GB-A- 1 277 002
- GB-A- 2 007 077
- GB-A- 2 516 491
- GB-A- 2 518 724
- NL-C2- 1 008 364
- US-A- 4 293 575
- WADE M A: "CALCIUM: THE CHOSEN FORM", PREPARED FOODS, GORMAN PUB., CHICAGO, IL, US, vol. 173, no. 5, 1 January 2004 (2004-01-01), pages 61 - 62, 64, 6, XP009077539, ISSN: 0747-2536

## Description

This invention relates to an edible formulation comprising edible fungal particles of a filamentous fungus and calcium ions, which can be for use as meat substitutes. The present invention also relates to a method of making an edible formulation.

It is known, for example from WO 00/15045 (DSM), WO96/21362 (Zeneca) and WO95/23843 (Zeneca) to use edible filamentous fungi as meat-substitutes, for example in the preparation of burgers and sausages. In such uses, filaments of the fungi are bound together, for example with egg albumin, and are texturised so that the product resembles muscle fibres and therefore has a meat-like appearance and texture. Meat substitutes of the type described have been widely commercially available for many years under the trade mark QUORN.

In some circumstances, it is desirable to reduce or even eliminate the amount of egg albumin used with edible fungus in the manufacture of meat-substitutes for example on cost grounds or to produce a product suitable for vegans.

It is an object of the present invention to address the aforementioned problem.

EP0986960A and EP1133926A describe a fermentation process for producing a food product based on the fungus Mucorales.

WO9955165 describes a skin which may include calcium ions as a hardening agent for the skin. The skin may enclose a proteinaceous composition which may comprise meat, meat substitutes, plant and/or fish products.

WO9117669 describes the use of calcium ions in the control of branching of filamentous microorganisms.

WO02089604 relates to edible fungi and provides a solid first food ingredient, the use thereof, a method of preparing a foodstuff and a foodstuff per se, especially an extruded foodstuff. It also provides methods and uses of edible fungi in the promotion of good health.

According to a first object aspect of the invention, there is provided an edible formulation as described in claim 1.

It follows from the aforementioned that said formulation includes 0 wt% of a hydrocolloid derived from an animal source; and said formulation includes 0 wt% of egg albumin.

Said edible formulation is preferably a meat substitute.

Said edible formulation includes at least 20 wt% of said filamentous fungus on a dry matter basis. In some cases, for example, when said edible formulation mimics a meat product (e.g. strips of meat or mince) which may be used as a food ingredient, said edible formulation may include higher levels of said filamentous fungus, for example greater than 40 wt% or greater than 60 wt% of filamentous fungus on a dry matter basis. Said edible formulation may include up to 80 wt%, preferably up to 75 wt% of filamentous fungus on a dry matter basis.

Said edible formulation suitably includes at least 6,000mg, especially at least 8,000mg, of calcium ions per Kg of filamentous fungus on a dry matter. Said edible formulation suitably includes less than 25,000mg, preferably less than 20,000mg, of said calcium ions per Kg of filamentous fungus on a dry matter basis.

Said edible formulation may include intracellular calcium ions (e.g. within the edible fungal particles) and extracellular calcium ions. Said edible formulation suitably includes the following extracellular levels of calcium ions: at least 2,000mg, preferably at least 4,000mg, more preferably at least 6,000mg, especially at least 8,000mg per Kg of filamentous fungus on a dry matter basis. The maximum extracellular level of calcium ions_is suitably less than 25,000mg, preferably less than 20,000mg, per Kg of filamentous fungus on a dry matter basis.

The total amount of calcium ions in said edible formulation is suitably at least 5,000mg per Kg, preferably at least 10,000mg per Kg, of filamentous fungus on a dry matter basis. The total amount of calcium ions may be less than 40,000mg or less than 20,000mg per Kg of filamentous fungus.

Said edible formulation may include at least 0.200 wt%, more preferably at least 0.300 wt%, of calcium ions in total, on a dry weight basis. It may include less than 0.8 wt% of calcium ions in total, on a dry weight basis.

Said edible formulation may include at least 60 wt%, water. Said formulation may include less than 85 wt% or less than 80 wt% water. Said edible formulation may include the aforementioned levels of water after a precursor of said edible formulation has been treated (e.g. cooked such as by steaming, chilling and/or freezing) to develop texture.

Said edible formulation may include a polysaccharide, for example a sulphonated polysaccharide, for example carrageenan. Said edible formulation, on a dry weight basis, suitably includes at least 0.1 wt%, preferably at least 0.5 wt%, more preferably at least 0.9 wt% of said polysaccharide, for example carrageenan. It may include less than 2 wt% of said polysaccharide.

Said edible formulation may include an alginate, for example a salt of alginic acid such as sodium alginate. Said edible formulation, on a dry weight basis, suitably includes 0.01 wt%, preferably at least 0.05 wt%, more preferably at least 0.12 wt% of alginate. It may include less than 1 wt%, preferably less than 0.5 wt% of alginate.

Said edible formulation may include gluten, for example wheat gluten. Said edible formulation, on a dry weight basis, suitably includes at least 0.1 wt%, preferably at least 1 wt%, more preferably at least 1.5 wt% of said gluten. It may include less than 5 wt% of said gluten.

Said edible formulation may include a protein source in addition to said edible fungal particles and which is not a wheat-based protein. Said protein source (A) may be a source of vegetable protein. Said protein source (A) may be a potato protein source. Said edible formulation, on a dry weight basis, may include at least 1 wt%, preferably at least 5 wt% of protein source (A). It may include less than 20 wt% of protein source (A).

It is found that, if calcium chloride alone is used in the formulation, even at a minimum level to achieve the effect described herein, a disagreeable taste may result. Accordingly, steps may be taken to counter the disagreeable taste. It has been found that use of calcium acetate is able to do this. Thus, said edible formulation includes acetate moieties (which may have been initially incorporated into the formulation as calcium acetate).

In said edible formulation, the ratio of the wt% of acetate ions divided by the wt% of filamentous fungus on a dry matter basis is suitably at least 0.005, preferably at least 0.01. Said ratio may be less than 0.04, for example less than 0.03.

Said acetate ions are suitably extra-cellular ions.

Said edible formulation may include at least 0.10 wt%, preferably at least 0.40 wt% of acetate ions on the dry matter basis. It may include less than 2.00 wt%, for example less than 1.00 wt% of acetate ions on a dry matter basis.

In a preferred embodiment, said edible formulation may include less than 25,000mg of calcium ions per Kg of filamentous fungus on a dry matter basis.

In preferred embodiments, preferably in said edible formulation, the ratio of the wt% of acetate ions divided by the wt% of filamentous fungus on a dry matter basis is at least 0.005 and is less than 0.03.

Said edible formulation preferably includes at least 0.10 wt%, preferably at least 0.50 wt%; and suitably includes less than 1.5 wt%, or less than 1.1 wt% of acetate ions, on a dry matter basis.

As described, fungal particles suitably comprise a filamentous fungus. Said filamentous fungus preferably comprises fungal mycelia and suitably at least 80 wt%, preferably at least 90 wt%, more preferably at least 95 wt% and, especially, at least 99 wt% of the fungal particles in said formulation comprise fungal mycelia. Some filamentous fungi may include both fungal mycelia and fruiting bodies. Said fungal particles preferably comprise a filamentous fungus of a type which does not produce fruiting bodies. Where, however, a filamentous fungus of a type which produces fruiting bodies is used, the fungal particles in said composition suitably include at least 80 wt%, preferably at least 90 wt%, more preferably at least 95 wt% of fungal mycelia. Preferably, said fungal particles comprise substantially only fungal mycelia - that is, said fungal particles in said composition preferably do not include any fruiting bodies.

Preferred fungi for said fungal particles have a cell wall which includes chitin and/or chitosan. Preferred fungi have a cell wall which includes polymeric glucosamine. Preferred fungi have a cell wall which includes β1-3 and 1-6 glucans.

Said fungal particles may include fungal cells of the order Mucorales as described in WO 00/15045 (DSM).

Said fungal particles preferably comprise fungus selected from fungi imperfecti.

Preferably, said fungal particles comprise, and preferably consist essentially of, cells of Fusarium species, especially of Fusarium venenatum A3/5 (formerly classified as Fusarium graminearum) (IMI 145425; ATCC PTA-2684 deposited with the American Type Culture Collection, 10801 University Boulevard, Manassas, VA.) as described for example in WO96/21361 (Zeneca) and WO95/23843 (Zeneca).

Preferably, said fungal particles are non-viable. Preferably, said fungal particles have been treated to lower the level of RNA which they contain. Thus, the level of RNA in the fungal particles used is preferably less than the level in an identical fungus when in a viable state. The level of RNA may be reduced as described in WO95/23843. Said fungal particles suitably have a RNA content on a dry weight basis of less than 1.9 wt%, for example 1.7 wt% or less.

Fungal particles in said formulation may comprise filaments having lengths of less than 1000 µm, preferably less than 800 µm. Preferably, fewer than 5 wt%, preferably substantially no, fungal particles in said formulation have lengths of greater than 5000µm; and preferably fewer than 5 wt %, preferably substantially no, fungal particles have lengths of greater than 2500 µm. Preferably, values for the number average of the lengths of said fungal particles in said formulation are also as stated above.

Fungal particles in said formulation may comprise filaments having diameters of less than 20 µm, preferably less than 10 µm, more preferably 5 µm or less. Said filaments may have diameters greater than 1 µm, preferably greater than 2 µm. Preferably, values for the number average of said diameters of said fungal particles in said formulation are also as stated above.

Fungal particles in said formulation may comprise filaments having an aspect ratio (length/diameter) of less than 1000, preferably less than 750, more preferably less than 500, especially of 250 or less. The aspect ratio may be greater than 70. Preferably, values for the average aspect ratio of said fungal particles (i.e. the average of the lengths of the particles divided by the average of the diameters of the fungal particles) in said formulation are also as stated above.

Said edible formulation may be provided in a alginate. Thus, said edible formulation is suitably surrounded by packaging material which may comprise a receptacle. Said package may include at least 100g of said edible formulation. Said package may include said edible formulation and include at least 10g of edible fungal particles on a dry matter basis. Said package may include an edible formulation which includes at least 20 wt%, for example at least 45 wt% water.

According to a second aspect of the invention, there is provided a method of making an edible formulation as described in the first aspect, the method comprising:
(i) selecting a formulation comprising edible fungal particles of a filamentous fungus;
(ii) contacting said formulation with calcium ions.

Contact may be effected so as to produce an edible formulation with calcium ions as described in the first aspect and/or at levels described in the first aspect.

Said edible fungal particles selected in step (i) are suitably fungal particles which have been treated after removal from a reactor in which the particles are produced in a fermentation process.

Said edible fungal particles selected in step (i) are preferably not viable.

Said edible fungal particles selected in step (i) preferably have been treated to lower the level of RNA they contain.

Said edible fungal particles selected in step (i) preferably have an RNA content on a dry weight basis of less than 1.9 wt%, for example 1.7 wt% or less.

Any feature of any aspect of any invention described herein may be combined with any other invention described herein mutatis mutandis.

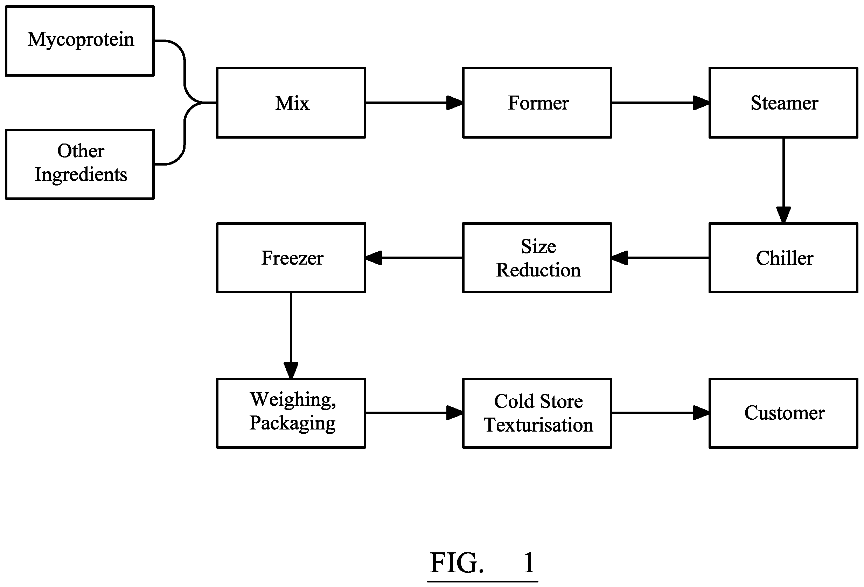

Specific embodiments of the invention will now be described, by way of example, with reference to the accompanying drawing, in which Figure 1 is a schematic representation of steps in the production of mycoprotein-containing products for human consumption.

The following materials are referred to hereinafter:
Mycoprotein paste - Mycoprotein paste-refers to a visco-elastic material comprising a mass of edible filamentous fungus derived from Fusarium venenatum A3/5 (formerly classified as Fusarium graminearum Schwabe) (IMI 145425; ATCC PTA-2684 deposited with the American type Culture Collection, 12301 Parklawn Drive, Rockville Md. 20852) and treated to reduce its RNA content to less than 2% by weight by heat treatment. Further details on the material are provided in WO96/21362 and WO95/23843. The material may be obtained from Marlow Foods Limited of Stokesley, U.K. It comprises about 23-25 wt % solids (the balance being water) made up of non-viable RNA reduced fungal hyphae of approximately 400-750 µm length, 3-5 µm in diameter and a branching frequency of 2-3 tips per hyphal length.

Calcium chloride solution - refers to a 36 wt% aqueous solution of calcium chloride.

Calcium acetate - refers to calcium acetate in solid form.

Sodium alginate refers to sodium alginate in a solid form.

In the examples which follow, Example 1 provides a general method of producing mycoprotein-containing products. Example 2 - 5 provide details of specific products which are suitable for consumption by vegans. It is found that the addition of calcium cations (via calcium chloride and calcium acetate) to the ingredients described in the examples produce a rise in firmness and produces acceptable quality of the product produced. It is believed the calcium cations interact with the mycoprotein paste to increase its firmness and strength.

### Example 1 - General process for preparation of products

This is summarized in Figure 1. Mycoprotein paste is mixed with other ingredients it is desired to incorporate to produce a substantially homogenous mass of a mycoprotein-containing foodstuff (for example meat-like pieces, mince, sausages and roast meats). The homogenous mass is put through a former and then a steamer (e.g. over 95°C for 35-45 minutes). The steamed product is then chilled (e.g. -5 to - 10°C for about 20 minutes) which improves the texture of the product by making it slightly firmer. There follows an optional size reduction process followed by a second texturization step involving freezing. Thereafter, products are weighed and packaged prior to the final texturization step at -18°C in a cold store for at least 7 days. Thereafter product can be delivered to retail outlets for sale to customers.

### Example 2 - Preparation of mycoprotein-containing pieces

Following the general procedure described in Example 1, the ingredients referred to in Table 1 were combined to produce the final product.

**Table 1**

| **Vegan Pieces recipe (Table 1)** | | |
|---|---|---|
| **Ingredient** | **Wet weight (g/kg)** | **Dry weight g/kg** |
| **Mycoprotein Paste** | **884.20** | **221.00** |
| **Water** | **12.00** | **<1.00** |
| **Flavour 1** | **10.00** | **9.50** |
| **Potato Protein** | **32.00** | **30.40** |
| **Vital Wheat Gluten** | **10.00** | **9.50** |
| **Calcium Acetate** | **4.00** | **3.80** |
| **Calcium Chloride Solution** | **12.00** | **4.30** |
| **Sodium Alginate** | **0.80** | **0.76** |
| **Flavour 2** | **3.00** | **2.85** |
| **Carageenan** | **4.00** | **3.80** |
| **Wheat Fibre** | **20.00** | **19.00** |
| **Pea Fibre** | **8.00** | **7.60** |
| **Total** | **1000.00** | **312.60** |

It should be appreciated that the steaming/chilling process affects the level of water in the final product which is generally in the range 70 to 77 wt% in total.

### Example 3 - Preparation of mycoprotein-containing mince

Following the general procedure described in Example 1, the ingredients referred to in Table 2 were combined to produce the final product.

**Table 2**

| **Vegan Mince recipe** | | |
|---|---|---|
| **Ingredient** | **Wet weight (g/kg)** | **Dry Weight(g/kg)** |
| **Mycoprotein Paste** | **888.20** | **222.00** |
| **Water** | **0.00** | **0.00** |
| **Potato Protein** | **50.00** | **47.50** |
| **Calcium acetate** | **4.00** | **3.80** |
| **Calcium Chloride Sodium** | **12.00** | **4.20** |
| **Sodium Alginate** | **0.80** | **0.76** |
| **Vital Wheat Gluten** | **10.00** | **9.50** |
| **Malt Extract** | **6.00** | **5.70** |
| **Caramelized Sugar** | **9.00** | **8.55** |
| **Wheat Fibre** | **14.00** | **13.30** |
| **Flavour** | **2.00** | **1.90** |
| **Carageenan** | **4.00** | **3.80** |
| **Total** | **1000.00** | **321.00** |

### Example 4 - Preparation of mycoprotein-containing burger

Following the general procedure described in Example 1, the ingredients referred to in Table 3 were combined to produce the final product.

**Table 3**

| **Vegan Burger Recipe** | | |
|---|---|---|
| **Ingredient** | **Wet Weight (g/kg)** | **Dry Weight (g/kg)** |
| **Mycoprotein Paste** | **385.20** | **176.60** |
| **Water** | **171.10** | **<1.00** |
| **Onions** | **100.00** | **15.00** |
| **Meatless Mince** | **83.30** | **16.70** |
| **Malt Extract** | **10.00** | **9.50** |
| **Texturized Wheat Protein** | **83.30** | **79.10** |
| **Oil** | **20.50** | **20.50** |
| **Flavour 1** | **30.00** | **28.50** |
| **Flaked Fat** | **38.88** | **36.90** |
| **Calcium Chloride Solution** | **3.90** | **1.40** |
| **Calcium Acetate** | **3.90** | **3.70** |
| **Flavour 2** | **3.00** | **2.90** |
| **Wheat Fibre** | **20.00** | **19.00** |
| **Carageenan** | **4.00** | **3.80** |
| **Sodium Alginate** | **0.80** | **0.76** |
| **Vital Wheat Gluten** | **10.00** | **9.50** |
| **Potato Protein** | **32.00** | **30.40** |
| **Total Weight** | **1000.00** | **455.00** |

### Example 5 - Preparation of mycoprotein-containing sausage

Following the general procedure described in Example 1, the ingredients referred to in Table 4 were combined to produce the final product.

**Table 4**

| **Vegan Sausage Recipe** | | |
|---|---|---|
| **Ingredient** | **Wet Weight (g/kg)** | **Dry Weight (g/kg)** |
| **Mycoprotein Paste** | **431.60** | **107.90** |
| **Water** | **170.00** | **<1.00** |
| **Oil** | **60.00** | **60.00** |
| **Onions** | **62.50** | **9.40** |
| **Pea Fibre** | **6.00** | **5.70** |
| **Textured Wheat Protein** | **37.50** | **35.60** |
| **Rusk** | **68.80** | **65.30** |
| **Meatless Mince** | **43.80** | **8.80** |
| **Tapioca Starch** | **10.00** | **9.50** |
| **Seasoning** | **31.20** | **29.70** |
| **Calcium Chloride Solution** | **4.40** | **1.40** |
| **Calcium Acetate** | **4.40** | **4.20** |
| **Flavour** | **3.00** | **2.80** |
| **Wheat Fibre** | **20.00** | **19.00** |
| **Carageenan** | **4.00** | **3.80** |
| **Sodium Alginate** | **0.80** | **0.76** |
| **Vital Wheat Gluten** | **10.00** | **9.50** |
| **Potato Protein** | **32.00** | **30.40** |
| **Total** | **1000.00** | **405.00** |

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. An edible formulation comprising edible fungal particles of a filamentous fungus and calcium ions, wherein said edible formulation includes 0 wt% of components derived from animals; and wherein said edible formulation includes at least 2,000mg of calcium ions per Kg of filamentous fungus on a dry matter basis;
wherein said edible formulation includes at least 20 wt% of said filamentous fungus on a dry matter basis;
wherein said edible formulation includes at least 0.100 wt% and less than 1 wt% of calcium ions in total, on a dry weight basis;
wherein said edible formulation includes at least 50 wt% of water;
wherein said edible formulation includes acetate moieties;
wherein said edible fungal particles comprise filaments with an aspect ratio (length/ diameter) of
greater than 40;
wherein said edible fungal particles have a number average length of greater than 200 µm; and
wherein said edible formulation is provided in a package, wherein said package includes at least 50g of said edible formulation.

2. A formulation according to claim 1, wherein said edible formulation includes at least 8,000mg of calcium ions per Kg of filamentous fungus on a dry matter basis.

3. A formulation according to claim 1 or claim 2, wherein said edible formulation includes at least 8,000mg, per Kg of filamentous fungus on a dry matter basis, of extracellular calcium ions.

4. A formulation according to any preceding claim, wherein the total amount of calcium ions in said edible formulation is at least 5,000mg per Kg; and may be less than 40,000mg per Kg of filamentous fungus.

5. A formulation according to any preceding claim, wherein said edible formulation includes:
(i) a polysaccharide, for example a sulphonated polysaccharide;
(ii) an alginate;
(iii) gluten; and/or
(iv) a protein source (A) in addition to said edible fungal particles, wherein said protein source (A) is a source of vegetable protein but is not a wheat-based protein.

6. A formulation according to any preceding claim, wherein in said edible formulation, the ratio of the wt% of acetate ions divided by the wt% of filamentous fungus on a dry matter basis is at least 0.005 and may be less than 0.04.

7. A formulation according to any preceding claim, wherein said edible formulation includes:
- 6,000mg of calcium ions per Kg of filamentous fungus on a dry matter basis;
- less than 25,000mg of calcium ions per Kg of filamentous fungus on a dry matter basis.

8. A formulation according to any preceding claim, wherein said fungal particles have a RNA content on a dry weight basis of less than 1.9 wt%.

9. A formulation according to any preceding claim, wherein in said edible formulation, the ratio of the wt% of filamentous fungus (on a dry matter basis) divided by the wt% of water is less than 0.5.

10. A formulation according to any preceding claim, wherein said filamentous fungus comprises fungal mycelia and at least 99 wt% of the fungal particles in said formulation comprise fungal mycelia.

11. A formulation according to any preceding claim, wherein said fungal particles comprise a filamentous fungus of a type which does not produce fruiting bodies.

12. A formulation according to any preceding claim, wherein said fungal particles consist essentially of cells of Fusarium species, especially of Fusarium venenatum A3/5.

13. A formulation according to any preceding claim, wherein said edible formulation is a meat substitute.

14. A formulation according to any preceding claim, wherein said package includes at least 100g of said edible formulation.

15. A method of making an edible formulation according to any preceding claim, the method comprising:
(i) selecting a formulation comprising edible fungal particles of a filamentous fungus;
(ii) contacting said formulation with calcium ions.

## Patentansprüche

1. Essbare Formulierung umfassend essbare Pilzpartikel eines Fadenpilzes und Calciumionen, wobei die essbare Formulierung 0 Gew.-% an von Tieren stammenden Komponenten enthält; und wobei die essbare Formulierung wenigstens 2.000 mg Calciumionen pro kg Fadenpilz auf Trockenmassebasis enthält;
wobei die essbare Formulierung wenigstens 20 Gew.-% an dem Fadenpilz auf Trockenmassebasis enthält;
wobei die essbare Formulierung insgesamt wenigstens insgesamt 0,100 Gew.-% und weniger als 1 Gew.-% Calciumionen auf Trockengewichtbasis enthält;
wobei die essbare Formulierung wenigstens 50 Gew.-% Wasser enthält;
wobei die essbare Formulierung Acetateinheiten enthält; wobei die essbaren Pilzpartikel Filamente mit einem Aspektverhältnis (Länge/Durchmesser) von größer als 40 umfassen;
wobei die essbaren Pilzpartikel eine anzahlgemittelte Länge von mehr als 200 µm aufweisen; und
wobei die essbare Formulierung in einer Verpackung bereitgestellt wird, wobei die Verpackung wenigstens 50 g an der essbaren Formulierung enthält.

2. Formulierung nach Anspruch 1, wobei die essbare Formulierung wenigstens 8.000 mg Calciumionen pro kg Fadenpilz auf Trockenmassebasis enthält.

3. Formulierung nach Anspruch 1 oder Anspruch 2, wobei die essbare Formulierung wenigstens 8.000 mg extrazelluläre Calciumionen pro kg Fadenpilz auf Trockenmassebasis enthält.

4. Formulierung nach einem der vorstehenden Ansprüche, wobei die Gesamtmenge an Calciumionen in der essbaren Formulierung wenigstens 5.000 mg pro kg beträgt und weniger als 40.000 mg pro kg Fadenpilz betragen kann.

5. Formulierung nach einem der vorstehenden Ansprüche, wobei die essbare Formulierung enthält:
(i) ein Polysaccharid, beispielsweise ein sulfoniertes Polysaccharid;
(ii) ein Alginat;
(iii) Gluten; und/oder
(iv) eine Proteinquelle (A) zusätzlich zu den essbaren Pilzpartikeln, wobei die Proteinquelle (A) eine Quelle von pflanzlichem Protein ist aber kein Protein auf Weizenbasis ist.

6. Formulierung nach einem der vorstehenden Ansprüche, wobei in der essbaren Formulierung das Verhältnis der Gew.-% an Acetationen geteilt durch die Gew.-% an Fadenpilz auf Trockenmassebasis wenigstens 0,005 beträgt und kleiner als 0,04 sein kann.

7. Formulierung nach einem der vorstehenden Ansprüche, wobei die essbare Formulierung enthält:
- 6.000 mg Calciumionen pro kg Fadenpilz auf Trockenmassebasis;
- weniger als 25.000 mg Calciumionen pro kg Fadenpilz auf Trockenmassebasis.

8. Formulierung nach einem der vorstehenden Ansprüche, wobei die Pilzpartikel einen RNA-Gehalt auf Trockengewichtsbasis von weniger als 1,9 Gew.-% aufweisen.

9. Formulierung nach einem der vorstehenden Ansprüche, wobei in der essbaren Formulierung das Verhältnis der Gew.-% an Fadenpilz (auf Trockenmassebasis) geteilt durch die Gew.-% Wasser kleiner als 0,5 ist.

10. Formulierung nach einem der vorstehenden Ansprüche, wobei der Fadenpilz Pilzmyzelien umfasst und wenigstens 99 Gew.-% der Pilzpartikel in der Formulierung Pilzmyzelien umfassen.

11. Formulierung nach einem der vorstehenden Ansprüche, wobei die Pilzpartikel einen Fadenpilz von einem Typ, der keine Fruchtkörper produziert, umfassen.

12. Formulierung nach einem der vorstehenden Ansprüche, wobei die Pilzpartikel im Wesentlichen aus Zellen von Fusarium-Spezies, insbesondere von Fusarium venenatum A3/5, bestehen.

13. Formulierung nach einem der vorstehenden Ansprüche, wobei die essbare Formulierung ein Fleischersatz ist.

14. Formulierung nach einem der vorstehenden Ansprüche, wobei die Verpackung wenigstens 100 g der essbaren Formulierung enthält.

15. Verfahren zur Herstellung einer essbaren Formulierung nach einem der vorstehenden Ansprüche, wobei das Verfahren umfasst:
(i) Auswählen einer Formulierung, die essbare Pilzpartikel eines Fadenpilzes umfasst;
(ii) Inkontaktbringen der Formulierung mit Calciumionen.

## Revendications

1. Formulation comestible comprenant des particules fongiques comestibles d'un champignon filamenteux et des ions calcium, dans laquelle ladite formulation comestible comprend 0 % en poids de composants dérivés d'animaux ; et dans laquelle ladite formulation comestible comprend au moins 2 000 mg d'ions calcium par kg de champignon filamenteux sur une base de matière sèche ;
dans laquelle ladite formulation comestible comprend au moins 20 % en poids dudit champignon filamenteux sur une base de matière sèche ;
dans laquelle ladite formulation comestible comprend au moins 0,100 % en poids et moins de 1 % en poids d'ions calcium au total, sur une base de poids sec ;
dans laquelle ladite formulation comestible comprend au moins 50 % en poids d'eau ;
dans laquelle ladite formulation comestible comprend des groupements acétate ;
dans laquelle lesdites particules fongiques comestibles comprennent des filaments ayant un rapport d'aspect (longueur/diamètre) supérieur à 40 ;
dans laquelle lesdites particules fongiques comestibles ont une longueur moyenne en nombre supérieure à 200 µm ; et
dans laquelle ladite formulation comestible est fournie dans un emballage, dans laquelle ledit emballage comprend au moins 50 g de ladite formulation comestible.

2. Formulation selon la revendication 1, dans laquelle ladite formulation comestible comprend au moins 8 000 mg d'ions calcium par kg de champignon filamenteux sur une base de matière sèche.

3. Formulation selon la revendication 1 ou la revendication 2, dans laquelle ladite formulation comestible comprend au moins 8 000 mg, par kg de champignon filamenteux sur une base de matière sèche, d'ions calcium extracellulaires.

4. Formulation selon l'une quelconque des revendications précédentes, dans laquelle la quantité totale d'ions calcium dans ladite formulation comestible est d'au moins 5 000 mg par kg ; et peut être inférieure à 40 000 mg par kg de champignon filamenteux.

5. Formulation selon l'une quelconque des revendications précédentes, dans laquelle ladite formulation comestible comprend :
(i) un polysaccharide, par exemple un polysaccharide sulfoné ;
(ii) un alginate ;
(iii) du gluten ; et/ou
(iv) une source de protéines (A) en plus desdites particules fongiques comestibles, dans laquelle ladite source de protéines (A) est une source de protéines végétales mais n'est pas une protéine à base de blé.

6. Formulation selon l'une quelconque des revendications précédentes, dans laquelle, dans ladite formulation comestible, le rapport du % en poids d'ions acétate divisé par le % en poids de champignon filamenteux sur une base de matière sèche est d'au moins 0,005 et peut être inférieur à 0,04.

7. Formulation selon l'une quelconque des revendications précédentes, dans laquelle ladite formulation comestible comprend :
- 6 000 mg d'ions calcium par kg de champignon filamenteux sur une base de matière sèche ;
- moins de 25 000 mg d'ions calcium par kg de champignon filamenteux sur une base de matière sèche.

8. Formulation selon l'une quelconque des revendications précédentes, dans laquelle lesdites particules fongiques ont une teneur en ARN sur une base de poids sec inférieure à 1,9 % en poids.

9. Formulation selon l'une quelconque des revendications précédentes, dans laquelle, dans ladite formulation comestible, le rapport du % en poids de champignon filamenteux (sur une base de matière sèche) divisé par le % en poids d'eau est inférieur à 0,5.

10. Formulation selon l'une quelconque des revendications précédentes, dans laquelle ledit champignon filamenteux comprend des mycéliums fongiques et au moins 99 % en poids des particules fongiques dans ladite formulation comprennent des mycéliums fongiques.

11. Formulation selon l'une quelconque des revendications précédentes, dans laquelle lesdites particules fongiques comprennent un champignon filamenteux d'un type qui ne produit pas de fructifications.

12. Formulation selon l'une quelconque des revendications précédentes, dans laquelle lesdites particules fongiques sont constituées essentiellement de cellules de l'espèce Fusarium, notamment de Fusarium venenatum A3/5.

13. Formulation selon l'une quelconque des revendications précédentes, dans laquelle ladite formulation comestible est un substitut de viande.

14. Formulation selon l'une quelconque des revendications précédentes, dans laquelle ledit emballage comprend au moins 100 g de ladite formulation comestible.

15. Procédé de préparation d'une formulation comestible selon l'une quelconque des revendications précédentes, le procédé comprenant :
(i) la sélection d'une formulation comprenant des particules fongiques comestibles d'un champignon filamenteux ;
(ii) la mise en contact de ladite formulation avec des ions calcium.
